# FASCICULE DE BREVET EUROPEEN

(11) **EP 2 414 379 B1**
(45) Date de publication et mention de la délivrance du brevet: **30.03.2016**
(21) Numéro de dépôt: 10717688.5
(22) Date de dépôt: 30.03.2010
(51) Int. Cl.: C07K 1/14, C12N 9/26

(54) **PROCÉDÉ D'OBTENTION D'UNE PRÉPARATION DE BÉTA-AMYLASES A PARTIR DES FRACTIONS SOLUBLES DE PLANTES AMIDONNIÈRES**
VERFAHREN ZUR GEWINNUNG EINER ZUBEREITUNG VON BETA-AMYLASEN AUS DEN LÖSLICHEN FRAKTIONEN VON STÄRKEPFLANZEN
METHOD FOR OBTAINING A PREPARATION OF BETA-AMYLASES FROM THE SOLUBLE FRACTIONS OF STARCH PLANTS

(30) Priorité: 30.03.2009 FR 0951962
(43) Date de publication de la demande: 08.02.2012
(73) Titulaire: Roquette Frères, 62136 Lestrem (FR)
(72) Inventeur: COURBOIS, Vincent, F-62400 Bethune (FR); DUFLOT, Pierrick, F-62136 La Couture (FR); PASSE, Damien, F-59500 Douai (FR)
(74) Mandataire: Gallois, Valérie
(86) Numéro de dépôt international: PCT/FR2010/050596
(87) Numéro de publication internationale: WO 2010/112765

(56) Documents cités:
- EP-A- 0 905 256
- JP-A- 7 107 974
- US-A- 4 675 296
- US-A- 5 294 341
- US-B2- 7 399 622
- UPADHYAY C M ET AL: "A PROTOCOL FOR OPTIMAL EXTRACTION AND STABILIZATION OF BARLEY BETA AMYLASE" BIOTECHNOLOGY TECHNIQUES, vol. 4, no. 2, 1990, pages 117-120, XP002546979 ISSN: 0951-208X
- BAJPAI P ET AL: "CLARIFICATION OF BACTERIAL BROTH CONTAINING HIGH ALPHA-AMYLASE ACTIVITY" BIOTECHNOLOGY TECHNIQUES, CHAPMAN & HALL, vol. 4, no. 4, 1 janvier 1990 (1990-01-01) , pages 227-232, XP008008107 ISSN: 0951-208X

## Description

La présente invention est relative à un procédé d'obtention d'une préparation de β-amylases à partir de résidus d'amidonnerie.

Plus particulièrement, la présente invention est relative à un procédé d'obtention d'une préparation de β-amylases à partir des résidus liquides (encore appelés « fractions solubles », quoique renfermant encore des substances insolubles résiduelles, des particules et des colloïdes divers et variés) produites lors de l'extraction par voie humide des composants (amidons, protéines et fibres) des plantes amidonnières.

Au sens de l'invention, on entend par « plantes amidonnières » des plantes susceptibles d'être traitées en amidonnerie pour en extraire l'amidon, telles notamment le maïs, la pomme de terre, la patate douce, le blé, le riz, le pois, la fève, la fèverole, le manioc, le sorgho, le konjac, le seigle, le sarrasin et l'orge. Dans un mode de réalisation particulier, les « plantes amidonnières » comprennent le maïs, la pomme de terre, le blé, le riz, le pois, la fève, la fèverole, le manioc, le sorgho, le konjac et l'orge.

La présente invention est également relative audit procédé comprenant une première étape de clarification des fractions solubles puis, de manière optionnelle, une deuxième étape d'ultrafiltration pour obtenir une préparation purifiée (au sens où elle est débarrassée de ses sels) et/ou concentrée en β-amylases. Dans un mode de réalisation préférée, la présente invention est également relative audit procédé comprenant une première étape de clarification des fractions solubles, une deuxième étape d'ultrafiltration puis une troisième étape de diafiltration pour obtenir une préparation purifiée (au sens où elle est appauvrie en sels) et concentrée en β-amylases.

Le procédé selon la présente invention permet enfin de disposer d'une préparation de β-amylases d'une qualité telle qu'il soit possible de l'utiliser de la même manière qu'une β-amylase purifiée, notamment pour la production de sirop riche en maltose.

Les P-amylases sont des exo-hydrolases qui libèrent des unités maltose à partir des extrémités non réductrices des polymères ou oligomères de glucose liés en α 1→4, la réaction s'arrêtant au premier point de branchement α 1→6 rencontré.

Composants majoritaires du « pouvoir diastatique » (correspondant aux activités combinées des α-amylases, β-amylases, α-glucosidases et enzymes débranchantes) durant le maltage (germination artificielle des graines de céréales), les activités β-amylasiques isolées de ce cocktail enzymatique sont essentielles pour la production du maltose ou d'autres sucres fermentescibles générés à partir d'amidon.

Le maltose et les autres sucres fermentescibles ainsi obtenus sont par exemple destinés à la fermentation éthanolique par les levures.

L'activité saccharifiante des seules β-amylases est donc exploitée dans bon nombre d'applications :
- en panification,
- en malterie,
- comme additif alimentaire, voire même comme agent « digestif »,
- pour la production de maltose et de sirops enrichis en maltose (précurseur du maltitol et des sirops de maltitol),
- pour la production d'édulcorants,
- en pharmacie, pour la production de vaccins.

Les P-amylases des céréales, des légumineuses et des tubercules sont toutes semblables, en regard de leur activité enzymatique.

Cependant, le fait que certaines plantes amidonnières soient choisies de préférence à d'autres comme sources de β-amylases pour la malterie, ou pour d'autres procédés biotechnologiques, est fonction de la richesse en β-amylases de leurs graines, et est fonction de la facilité avec laquelle elles peuvent être isolées de ces milieux riches en activités enzymatiques variées.

C'est ainsi que deux sources d'extraction des β-amylases sont bien identifiées.

La première source d'extraction des β-amylases dites « classiques » est l'endosperme du blé, de l'orge ou du seigle, source connue pour produire les β-amylases de façon majoritaire et en grandes quantités.

La seconde source d'extraction correspond aux graines mises artificiellement à germer. Il est ainsi procédé à un enrichissement artificiel notamment en β-amylases par le déclenchement de la germination ; les activités β**-**amylasiques sont alors surexprimées avec d'autres activités amylasiques et glucosidasiques.

Le domaine technique auquel s'attache la présente invention est celui de la première source d'extraction, permettant de produire plus efficacement la β-amylase en tant que telle, en quantité plus importante. Le domaine technique de la présente invention concerne également les fractions solubles résiduelles issues du traitement en amidonnerie de plantes telles que le blé, l'orgue, le seigle, le maïs, le sorgho, le sarrasin, le riz, le pois, la fève, la fèverole, la pomme de terre, le manioc, la patate douce, le konjac. La seconde source de β-amylases étant plus particulièrement destinée à la préparation de cocktails enzymatiques.

Il est déjà admis de l'homme du métier que les graines d'orge, de seigle ou de blé non germées sont autant de matériels biologiques de choix pour la préparation commerciale, à grande échelle, de β-amylases.

Il est par ailleurs connu de l'homme du métier que la moitié des P-amylases extractibles des graines non germées de l'orge, du blé ou du seigle peut être facilement obtenue sous la forme d'enzymes libres par extraction avec de l'eau et des solutions salines.

L'autre moitié se présente en partie sous forme « liée » qui nécessite l'addition d'agents réducteurs ou des enzymes protéolytiques pour son extraction.

Une autre fraction de β-amylases non directement extractible, dite « latente » a également été décrite : il faut des détergents pour l'extraire des graines de céréales.

Par ailleurs, les procédés d'extraction de la β-amylase décrits dans l'état de la technique sont adaptés en fonction de l'application visée.

Plus particulièrement celles destinées à la saccharification de l'amidon et pour les essais expérimentaux sont extraites de façon intensive de sources botaniques, telles que le soja, le blé, l'orge, l'avoine, le seigle et la patate douce, ou sont produites par des microorganismes, tels que *Bacillus polymixa* et autres.

Dans le cas de la préparation du maltose à partir d'amidon, la première contrainte est de limiter au maximum la production concomitante d'autres oligosaccharides tels le glucose et maltotriose. La seconde contrainte consiste à utiliser une préparation de β-amylases substantiellement exempte de mycotoxines, de sels, de protéines et peptides de bas poids moléculaires (inférieures à 50 kDa et préférentiellement inférieures à 30 kDa), non allergène (en particulier grâce à l'absence de bisulfite dans le procédé de préparation) afin de permettre une purification (filtration, décoloration, déminéralisation) rapide et aisée du sirop riche en maltose et d'obtenir un sirop plus stable.

En malterie, par exemple, il est connu que la présence du glucose perturbe la fermentation des levures en inhibant leur croissance.

Pour la fabrication commerciale de maltitol à partir des sirops de maltose, il est recommandé de limiter la production de sorbitol et maltotriitol.

Il sera donc préféré de l'homme du métier les procédés permettant d'obtenir des préparations de β-amylases les plus pures possibles. Ces procédés auront bien évidemment l'inconvénient d'être coûteux.

Dans le brevet US 3.492.203, il est ainsi décrit l'extraction de β-amylases pures à partir du son de blé pour la préparation de sirop riche en maltose.

Il est ainsi évité de partir du malt, qui contient également de l'α-amylase, difficile à séparer de la β-amylase.

Le son de blé présente alors l'avantage d'être peu coûteux, recyclable comme milieu de culture, mais est surtout choisi pour son contenu majoritaire en β-amylases.

Le procédé consiste ici à extraire la β-amylase pure avec de l'eau à une température comprise entre 20 et 40°C

Cependant, ce procédé ne permet de récupérer efficacement que la moitié du contenu en β-amylase disponible du son.

Par ailleurs, l'inconvénient majeur de ce procédé est qu'il fournit une β-amylase instable bactériologiquement : la β-amylase ainsi produite doit être utilisée extemporanément pour saccharifier l'amidon. Aucune pasteurisation n'est réalisée.

Dans le brevet US 3.801.462, la β-amylase est extraite de la patate douce, plus particulièrement de l'eau de lavage après l'étape de râpage des patates douces.

Le procédé proprement dit d'extraction consiste en un traitement à un pH de 4-4,5 et la récupération de la β-amylase sous forme d'un précipité. Le rendement d'extraction est plus élevé lorsque la température est ajustée à 55°C.

Cependant, des activités α-amylasiques et maltases contaminent encore cette fraction enrichie en β-amylases, ce qui complique bien évidemment l'obtention de préparation pure.

Dans le brevet US 4.675.296, il est proposé une méthode de préparation d'une β-amylase commerciale par un procédé d'extraction à l'eau (à une température comprise entre 5 et 50°C, pendant 5 à 70 h), mais qui demande de partir de grain d'orge intact ou partiellement décortiqué.

Le procédé décrit est délicat à mettre en oeuvre, car il repose sur l'utilisation de la surface du grain comme une membrane semi-perméable, qui retiendrait tous les constituants dudit grain, sauf la β-amylase.

Il est cependant utile d'ajouter un agent réducteur afin de faciliter la libération de la β-amylase, agent réducteur à base de dioxyde de soufre ou d'acide sulfurique.

Par ailleurs, il est recommandé de stabiliser l'enzyme par des sels, des polyols, des sucres ou des acides.

D'autres procédés de la littérature recommandent d'utiliser des enzymes afin de faciliter l'extraction des β-amylases.

Dans la demande de brevet internationale WO 02/062980, il est ainsi procédé à l'extraction en milieu aqueux de β-amylases à partir de céréales en présence d'un cocktail complexe d'enzymes présentant des activités de cellulases, d'hémicellulases et de β-glucanases. L'extrait obtenu doit être finement purifié afin de séparer la β-amylase ainsi récupérée du mélange réactionnel.

Les céréales choisies sont l'orge et le blé.

L'extraction est effectuée en conditions réductrices (eau sulfitée) dans des proportions bien définies, dans des gammes de température et de temps de réaction bien arrêtées et à pH 6,5.

Dans l'US 3.769.168, il est préféré la voie de la purification d'extraits enrichis en β-amylases.

Les extraits bruts préparés à partir de son de blé, de soja ou de patates douces sont traités avec un agent adsorbant de type bentonite ou kaolinite.

Les β-amylases adsorbées sont ensuite éluées à l'aide d'une solution présentant une force ionique de plus de 0,5 µ à un pH supérieur à 5.

De tout ce qui précède, il résulte qu'il demeure un besoin non satisfait de disposer d'un procédé simple et peu couteux permettant d'obtenir des préparations de β-amylases de qualité suffisante pour permettent notamment la production efficace de sirops riche en maltose.

L'invention a donc pour but de répondre à ce besoin et la société Demanderesse a eu le mérite de trouver, après de nombreux travaux, que ce but pouvait être atteint contre toute attente dès lors que l'on utilise les résidus d'amidonnerie, en particulier les résidus liquides d'amidonnerie (encore appelés « fractions solubles », quoique renfermant encore des substances insolubles résiduelles, des particules et des colloïdes divers et variés).

Plus particulièrement, le procédé d'obtention de la préparation de β-amylases conforme à l'invention consiste à choisir les milieux sources de β-amylases dans le groupe constitué des fractions solubles du blé, du pois, de la fève, de la fèverole, du riz, de l'orge, du seigle, du sarrasin, de la patate douce et de la pomme de terre, préférentiellement les fractions solubles du blé, du pois, de la fève, de la fèverole, du riz, de l'orge et de la pomme de terre, plus préférentiellement les fractions solubles du blé et de l'orge, à clarifier lesdits milieux puis, à effectuer une ultrafiltration afin de récupérer, voire de concentrer, les β-amylases qu'elles contiennent.

De préférence, le procédé d'obtention de la préparation de P-amylases conforme à l'invention consiste à choisir les milieux sources de β-amylases dans le groupe constitué des fractions solubles du blé, du pois, de la fève, de la fèverole, du riz, de l'orge, du seigle, du sarrasin, de la pomme de terre et de la patate douce, préférentiellement les fractions solubles du blé et de l'orge, à clarifier lesdits milieux puis à effectuer une ultrafiltration et une diafiltration afin de récupérer et de concentrer les β-amylases qu'elles contiennent. Au cours du procédé d'obtention de la préparation de β-amylases conforme à l'invention aucune rectification de pH n'est effectuée, le pH est naturellement de l'ordre de 4,5.

Il est du mérite de la société Demanderesse d'avoir trouvé que les résidus d'amidonnerie peu valorisables dans des secteurs autres que celui de la fermentation (comme source azotée), ou celui de la nutrition animale (mélangés avec les issues du blé) pouvaient constituer un milieu de choix pour extraire aisément des enzymes d'intérêt, et plus particulièrement les β-amylases, à un niveau de qualité suffisante pour les applications visées, telle la préparation de sirop riche en maltose.

Le procédé conforme à l'invention est caractérisé en ce que l'on :
a) choisit la fraction soluble des plantes amidonnières dans le groupe constitué des fractions solubles du blé, du pois, de la fève, de la fèverole, du riz, de l'orge, du seigle, du sarrasin, de la patate douce et de la pomme de terre, préférentiellement du blé, de la pomme de terre, du pois, de fève, de fèverole, du riz, de l'orge et plus préférentiellement du blé et de l'orge,
b) effectue une clarification desdites fractions solubles de manière à en éliminer les substances insolubles et les colloïdes,
c) réalise une ultrafiltration desdites fractions solubles clarifiées de manière à obtenir un rétentat d'ultrafiltration contenant la β-amylase concentrée et un perméat d'ultrafiltration,
d) récupère la β-amylase concentrée ainsi obtenue.

De préférence, le procédé conforme à l'invention est caractérisé en ce que l'on :
a) choisit la fraction soluble des plantes amidonnières dans le groupe constitué des fractions solubles du blé, de la pomme de terre, du pois, de fève, de fèverole, du riz, de l'orge, du seigle, du sarrasin, de la patate douce et préférentiellement du blé et de l'orge,
b) effectue une clarification desdites fractions solubles de manière à en éliminer notamment les substances insolubles, les colloïdes, le matériel microbiologique,
c) réalise une ultrafiltration desdites fractions solubles clarifiées de manière à obtenir un rétentat d'ultrafiltration contenant la β-amylase concentrée et un perméat d'ultrafiltration (encore appelé filtrat),
d) réalise une diafiltration dudit rétentat d'ultrafiltration contenant la β-amylase concentrée,
e) récupère la β-amylase concentrée ainsi obtenue.

Il peut être également réalisé le mélange du perméat d'ultrafiltration avec les insolubles et colloïdes de l'étape b).

Le procédé conforme à l'invention implique donc uniquement l'association de techniques d'extractions physiques telles que des techniques de filtration ou centrifugation et n'implique aucune étape d'extraction enzymatique. Il n'y a donc aucun besoin de la présence d'un cocktail enzymatique contenant de la cellulase, de l'hémicellulase et/ou de la β-glucanase. Il n'y a aucun besoin également d'effectuer une rectification de pH.

La première étape du procédé conforme à l'invention consiste à choisir la fraction soluble des plantes amidonnières dans le groupe constitué des fractions solubles du blé, de la pomme de terre, du pois, de fève, de fèverole, du riz, de l'orge, du seigle, du sarrasin, de la patate douce, préférentiellement du blé, de la pomme de terre, du pois, de fève, de fèverole, du riz, de l'orge et plus préférentiellement du blé et de l'orge.

Dans la présente invention, on entend par « fraction soluble » les eaux résiduelles produites lors de l'extraction par voie humide des composants nobles des plantes amidonnières. On entend ici par « composants nobles » les composants valorisés en amidonnerie classique, en particulier, et en fonction de la plante amidonnière, l'amidon quel qu'il soit, les protéines, les fibres, les gommes. Ladite fraction soluble selon l'invention ne comprend donc pas ou substantiellement pas ces dits composants nobles. C'est pour cette raison que ces fractions solubles sont appelées également eaux résiduelles ou encore résidus liquides.

Les fractions solubles ou solubles de blé, par exemple, proviennent du flux de séparation des amidons de blé « B » résultant de la séparation de l'amidon dans le procédé de l'amidonnerie de blé humide. L'amidon B ou amidon second est l'amidon constitué essentiellement par une proportion prépondérante de petits granules d'amidon ou de granules endommagés. Selon la présente invention, les solubles de blé constitués des eaux de raffinage de l'amidon présentent généralement environ 5% de matière sèche, en particulier moins de 8% de matière sèche.

Les fractions solubles ou solubles de pomme de terre, de la patate douce ou du manioc sont obtenues par récupération de la fraction soluble issue de l'écrasage des tubercules en début d'extraction de la fécule.

Les fractions solubles ou solubles de pois résultent de l'eau de trempage du pois et sont récupérées avant l'écrasage et la séparation des différents constituants du pois.

Les fractions solubles ou solubles de fève résultent de l'eau de trempage des fèves et sont récupérées avant l'écrasage et la séparation des différents constituants de la fève.

Les fractions solubles ou solubles de fèverole résultent de l'eau de trempage des fèveroles et sont récupérées avant l'écrasage et la séparation des différents constituants de la fèverole

Les fractions solubles ou solubles de riz résultent de l'eau de trempage du riz et sont récupérées avant l'écrasage et la séparation des différents constituants du riz.

Les fractions solubles ou solubles d'orge, de seigle ou de sarrasin sont issues du flux de séparation des amidons « B » résultant de la séparation de l'amidon dans le procédé de l'amidonnerie d'orge. L'amidon B ou amidon second est l'amidon constitué essentiellement par une proportion prépondérante de petits granules d'amidon ou de granules endommagés.

Ces fractions solubles renferment encore des protéines de haut poids moléculaire, dont certaines présentent des activités enzymatiques d'intérêt, cependant mélangées à des débris insolubles de toute sorte (notamment fibres, traces d'amidon...), des substances colloïdales (notamment phospholipides, glycoprotéines, cellules de l'assise externe de l'endosperme, encore appelée la couche d'aleurone...) et le matériel microbiologique (germes, bactéries...).

La société Demanderesse a donc bien été à l'encontre d'un préjugé de l'état de la technique qui ne décrivait jusqu'à présent l'utilisation par l'homme du métier de ces fractions solubles que dans deux domaines d'application à faible valeur ajoutée :
- source d'azote en fermentation, après qu'un traitement protéasique ait rendu la composante protéique de haut poids moléculaire résiduelle assimilable par les microorganismes,
- source nutritive pour les animaux d'élevage, et encore adjuvantée de matières fibreuses (e.g. son de blé dans le cas des solubles de blé).

La deuxième étape du procédé conforme à l'invention consiste donc à réaliser une étape de clarification desdites fractions solubles de manière à en éliminer les substances insolubles et les colloïdes.

Cette étape de clarification est contrôlée par la mesure de la turbidité de la solution après chacun des traitements effectués.

La turbidité désigne la teneur d'un liquide en matières qui le troublent. Elle est causée par des particules colloïdales qui absorbent, diffusent et/ou réfléchissent la lumière.

Cette mesure de turbidité est réalisée à l'aide d'un néphélomètre (ou turbidimètre) de laboratoire.

Cette méthode est normalisé (NF EN ISO 7027) ; deux unités de mesure de la turbidité utilisant la formazine comme étalon :
- FNU (Formazine Néphélométric Unit), ou NFU utilisé dans le décret n°2001-1220 du 20 décembre 2001. Cette unité mesure la turbidité sous un angle de 90° à une longueur d'onde de 860 nm ;
- FAU (Formazine Atténuation Unit) mesure la lumière transmise (180°).

L'unité de turbidité prescrite par l'Environmental Protection Agency (EPA - USA) est le NTU (Nephelometric Turbidity Unit). La mesure s'effectue sur la lumière diffusée à 90°, mais à une longueur d'onde différente de 860 nm.

Quant à la mesure de l'activité enzymatique, elle consiste à déterminer l'activité diastasique, mesure classiquement utilisée pour déterminer l'activité amylasique du malt de l'orge ou d'autres préparations enzymatique (cf article DIASTASE ACTIVITY - DIASTATIC POWER - de la partie General Tests and Assay / Appendix V / pp 1117-1118 du Food Chemical Codex 6).

L'activité diastasique est exprimée en degré de pouvoir diastasique (°DP), défini comme la quantité d'enzyme contenue dans 0,5 ml d'une solution à 5 % d'un échantillon de préparation d'enzymes suffisante pour réduire 5 ml de liqueur de Fehling, quand ledit échantillon est placé dans 100 ml du substrat pendant 1 h à 20°C.

Le pouvoir diastasique permettant de mesurer toutes les activités enzymatiques de type amylasique, la société Demanderesse s'est assurée par d'autres méthodes enzymatiques plus spécifiques (par exemple en utilisant le kit de dosage MEGAZYME spécifique à l'α-amylase commercialisé par CERALPHA METHOD) que la préparation de β-amylases conforme à l'invention ne refermait pas d'autres activités contaminantes.

De même, comme il sera exemplifié ci-après, les essais de saccharification en laboratoire avec la préparation enzymatique conforme à l'invention ont montré une forte teneur en maltose sans présence significative de glucose.

La première étape de clarification du procédé conforme à l'invention peut être réalisée de trois façons différentes :
1) Dans un premier mode de réalisation du procédé conforme l'invention, la clarification des fractions solubles est réalisée par filtration, au moyen d'une technique choisie dans le groupe constitué de la filtration frontale, de la filtration sous pression et de la filtration sous vide.

Les fractions solubles de départ présentent une turbidité élevée, dont la mesure ne peut être faite étant donné leur forte charge en particules colloïdales et insolubles résiduels.

Dans le cas de l'utilisation d'un filtre sous pression, ces fractions solubles sont poussées à travers ce filtre préalablement chargé de cellulose microcristalline. Le produit sortant du filtre présente alors une turbidité de l'ordre de 20 à 60 NTU selon le type de cellulose utilisé.

Dans le cas de la filtration sous vide, la société Demanderesse recommande d'effectuer la filtration à l'aide de filtres à bandes ou de filtres rotatifs.

On utilise par exemple un filtre à précouche sous vide préalablement chargé d'une couche de cellulose.
2) Dans un deuxième mode de réalisation du procédé conforme l'invention, la clarification des fractions solubles est réalisée par centrifugation.

Le surnageant de centrifugation des solubles présente alors également une turbidité de 20 à 60 NTU selon le débit et la concentration du culot de centrifugation (plus l'accélération de la centrifugeuse sera élevée, plus le produit sortant dans le surnageant aura une faible turbidité).

Plus particulièrement, la société Demanderesse recommande de réaliser cette étape de centrifugation à l'aide :
- d'une centrifugeuse à assiettes de type séparateur à buses ou de type auto-débourbeur NA7, ou
- d'une centrifugeuse à assiettes de type séparateur à buses vortex haute performance BTUX (ces deux types de centrifugeuse NA7 ou BTUX étant respectivement commercialisées par la société Westfalia et AlfaLaval).

Après centrifugation, mais de manière facultative, il peut être utile de procéder à une filtration supplémentaire dite de « sécurité » en vue de diminuer au maximum la charge des fractions solubles. Le filtre choisi est un filtre de type PADOVAN^{®} à plateaux horizontaux sous pression.

Le filtre est chargé avec une précouche de cellulose de fine granulométrie.

Les fractions solubles ainsi filtrées sont adjuvantées par un nourrissage plus ou moins important avec de la cellulose.

La turbidité du filtrat tombe ainsi à une valeur comprise entre 5 et 10 NTU.
3) Dans un troisième mode de réalisation du procédé conforme l'invention, la clarification des fractions solubles est réalisée par filtration tangentielle membranaire.

Plus particulièrement, la société Demanderesse recommande de réaliser la filtration membranaire par microfiltration tangentielle avec des membranes par exemple céramiques présentant une porosité de 0,1 à 1 µm.

Le perméat dans ce cas présente avantageusement une turbidité de l'ordre de 0 NTU.

Cette technique peut conduire cependant à une perte de β-amylases qui s'adsorbe à la membrane. Cependant, cette perte est négligeable en regard de la quantité présente initialement dans les fractions solubles.

Cette étape de clarification du procédé conforme à l'invention peut consister en l'un quelconque de ces trois modes de réalisation (filtration, centrifugation ou filtration tangentielle membranaire) ou en une association quelconque de deux ou trois de ces modes de réalisation.

De manière facultative, cette étape de clarification peut être précédée d'une étape de floculation des particules colloïdales, par toute technique connue par ailleurs de l'homme du métier, comme il sera exemplifié ci-après.

Pour disposer d'une préparation débarrassée des sels résiduels contaminants et pour concentrer ladite préparation en β-amylases, on réalise une ultrafiltration desdites fractions solubles clarifiées de manière à obtenir un rétentat d'ultrafiltration contenant la β-amylase et un perméat d'ultrafiltration. Le rétentat d'ultrafiltration est alors dialysé à volume constant de façon à diminuer la concentration en impuretés dans ledit rétentat.

Plus particulièrement, la société Demanderesse recommande de réaliser l'ultrafiltration à l'aide de membrane présentant un seuil de coupure de 10 000 Da à 50 000 Da, de préférence un seuil de 30 000 Da (noté également 30 kDa).

Les fractions solubles sont ultrafiltrées sur un module équipé de membranes polysulfonées d'un seuil de coupure de 30 000 Da en cassettes à l'échelle laboratoire et membranes spiralées polysulfonées d'un seuil de coupure de 30 000 Da à l'échelle pilote.

L'enzyme se concentre dans le rétentat au fur et à mesure qu'augmente le Facteur de Concentration Volumique (FCV).

Pour dix litres d'une solution de départ dosée à 22 °DP/ml, on obtient pour un FCV de 5, deux litres de rétentat à 110 °DP/ml et huit litres de perméat ne présentant pas de pouvoir diastasique.

Le rétentat d'ultrafiltration est diafiltré à volume constant, selon le procédé conforme à l'invention. En effet, l'obtention de macromolécules à haut degré de pureté nécessite la mise en oeuvre d'une telle étape de diafiltration afin d'épuiser (par dilution et perméation) le rétentat d'ultrafiltration en solutés non retenus par la membrane.

Une dernière étape du procédé conforme à l'invention peut consister à mélanger le perméat d'ultrafiltration avec les insolubles et colloïdes de l'étape b). Ce mélange rejoint les fractions solubles non traitées de l'amidonnerie.

Comme il sera exemplifié, et de manière surprenante et inattendue, la préparation ainsi préparée présente une qualité telle qu'il est possible de l'utiliser en tant que telle, sans traitement complémentaire, comme source d'enzymes destinées à la préparation de sirops riches en maltose.

### Exemple 1 : Obtention d'une première préparation de β-amylases à partir de solubles de blé - Echelle laboratoire

En amidonnerie de blé, on prélève 60 litres de fractions solubles à l'entrée de l'évaporateur des solubles, étape classiquement mise en oeuvre pour l'alimentation du bétail après concentration, commercialisé par la société Demanderesse sous le nom de CORAMI®.

Ces fractions solubles présentent un pH de 4 et une activité β-amylasique de l'ordre de 25 °DP/ml.

On centrifuge ces 60 litres sur centrifugeuse à assiettes de type débourbeuse SA1 commercialisée par la société WESTFALIA, à un débit de 40 litres/heure.

45 litres de surnageant de la centrifugation sont récupérés. On mesure une turbidité de 60 NTU.

On les filtre ensuite sur un filtre à plaque chambrée de laboratoire de type CHOQUENET de 56 cm², filtre à toiles préalablement chargé avec des celluloses de type CLAR O CEL 13/6 (CECA) et VITACEL L10 (J.RETTENMAIER UND SÖHNE).

On effectue également un nourrissage continu avec ces celluloses à raison de 1g/l.

On récupère 40 litres de filtrat d'une turbidité de 8 NTU et d'une activité β-amylasique de 22 °DP/ml.

Le filtrat est ensuite ultrafilté sur un module de laboratoire MILLIPORE avec 0,18 m² de membranes.

On récupère 39,5 litres de filtrat ultrafiltré et un rétentat concentré d'un facteur 75, présentant une activité β-amylasique comprise entre 1500 et 1600 °DP/ml.

On dialyse le rétentat d'ultrafiltration à volume constant avec 2,5 volumes d'eau en continu de façon à diminuer la concentration en impuretés des solubles d'un facteur de 10.

La préparation de β-amylases ainsi obtenue est alors stockée à + 4°C.

### Exemple 2 : Obtention d'une première préparation de β-amylases à partir de solubles de blé - Echelle laboratoire avec floculant

En amidonnerie de blé, on prélève 120 litres de fractions solubles à l'entrée de l'évaporateur des solubles à un pH de 4,3.

On traite ensuite avec 20 ppm de Fe³⁺ et 25 ppm de floculant anionique FLOPAM AN 923 PWG (SNF) de type polymérique de manière à floculer les particules insolubles et colloïdales.

Les fractions solubles ainsi traitées sont ensuite centrifugées dans les conditions de l'exemple 1 à un débit de 40 litres /heure.

90 litres de fractions solubles ainsi centrifugés sont obtenus. Ils présentent une turbidité de l'ordre de 17 NTU et une activité β-amylasique est de 21 °DP/ml.

De la même manière que dans l'exemple 1, cette fraction subit une filtration fine sur filtre à plaque chambrée.

On obtient 80 litres d'une solution filtrée présentant une turbidité de 5 NTU.

On ultrafiltre ensuite sur un module de laboratoire MILLIPORE avec 0,18m² de membranes d'un seuil de coupure de 30 kDa.

On récupère 79 litres de filtrat ultrafiltré et un rétentat concentré d'un facteur 80 et présentant une activité β-amylasique comprise entre 1500 et 1600 °DP/ml.

On dialyse le rétentat d'ultrafiltration à volume constant avec 2,5 volumes d'eau en continu de façon à diminuer la concentration en impuretés des solubles d'un facteur de 10.

La préparation de β-amylases ainsi obtenue est alors stockée à + 4°C.

### Exemple 3 : Obtention d'une première préparation de β-amylases à partir de solubles de blé - Echelle Pilote

L'essai Pilote consiste en un essai où 10 m³ de fractions solubles de blé sont prélevées et préparées comme dans l'exemple 2.

Les particules insolubles et colloïdales sont éliminées par centrifugation sur un équipement de type NA7 en mode de fonctionnement auto débourbage.

On récupère 7 m³ de surnageant présentant une turbidité de 22 NTU.

Ce surnageant est en ensuite filtré par filtration frontale sur un filtre AMAFILTER 0,3 m² chargé avec les celluloses préalablement définies. On le nourrit avec 1 g/l de cellulose.

Une fois filtrée, la solution présente une turbidité de 5 NTU.

On l'ultrafiltre sur un équipement constitué de membranes spirales polysulfonées d'un seuil de coupure de 30 kDa.

On obtient alors 75 litres de rétentat concentré d'un facteur 95 environ et présentant une activité β-amylasique d'environ 2000 °DP/ml.

On dialyse le rétentat d'ultrafiltration à volume constant avec 2,5 volumes d'eau en continu de façon à diminuer la concentration en impuretés des solubles d'un facteur de 10.

Pour stabiliser l'enzyme, on ajoute 50 Kg de sorbitol poudre commercialisés par la société Demanderesse sous le nom de marque NEOSORB^{®} à 99.5 % de matière sèche.

La préparation finale présente alors une activité β-amylasique de 1550 °DP/ml.

### Exemple 4 : Obtention de la préparation de β-amylases à partir de solubles d'orge

On ajoute dans un pétrin 100 kg de farine d'orge et 72 litres d'eau de façon à obtenir une pâte dure.

Après 30 minutes de repos à température ambiante, la pâte est lavée à contre-courant dans un tamis rotatif réalisé par la société Demanderesse avec de l'eau de manière à séparer l'amidon du gluten.

On recueille 200 litres d'une suspension d'amidon que l'on tamise sur un premier tamis vibrant de 100 µm, puis un second de 63 µm, pour éliminer les fibres, les gommes et le gluten résiduel.

Le filtrat est ensuite centrifugé à fort débit (200 l/h) sur un décanteur centrifuge commercialisé sous le nom de SEDICANTEUR par la marque FLOTTWEG de manière à isoler les amidons A (gros granule) dans le concentrat et les amidons B (petit granule) dans le surnageant.

L'overflow de suspension d'amidon B est à nouveau centrifugé sur la séparatrice à assiette SA1 (Westfalia) débourbeuse à débit réduit (40 L/h) de manière à isoler l'amidon B, dans le concentrat, et de la fraction soluble, dans le surnageant. Le pH est de 4,5.

Toutes ces étapes préalables permettent d'obtenir une fraction soluble conforme à la présente invention, i.e. débarrassée des composants nobles, en particulier, débarrassée l'amidon quel qu'il soit, les protéines, les fibres et les gommes. On prélève 60 litres de ladite fraction soluble qui présente une turbidité de 120 NTU.

On traite la fraction soluble selon le procédé de floculation de l'exemple n°2.

On centrifuge sur la centrifugeuse débourbeuse précédente à 40L/h et on récupère 40 litres de fraction soluble présentant une activité β-amylasique de 28 °DP/ml et une turbidité de 21 NTU.

Cette fraction soluble est filtrée sur un filtre à plaque chambrée préalablement chargé avec des celluloses de type CLAR O CEL 13/6 (CECA) et VITACEL L10 (J.RETTENMAIER UND SÖHNE).

La fraction soluble a été nourrie avec 1 g/l de celluloses. 40 litres de filtrat présentant une turbidité de 6 NTU et une activité β-amylasique de 27 °DP/ml sont ainsi obtenus.

On ultrafiltre sur module de laboratoire MILLIPORE avec 0,18m² de membranes 30 KDa, décrit dans l'exemple 2.

On effectue une concentration d'un facteur 65 environ.

On obtient finalement 0,6 litres d'une solution d'enzymes concentrées présentant une activité β-amylasique de 1700 °DP/ml.

On dialyse le rétentat d'ultrafiltration à volume constant avec 2,5 volumes d'eau en continu de façon à diminuer la concentration en impuretés des solubles d'un facteur de 10.

On la stabilise avec 400 g de sorbitol poudre commercialisés par la société Demanderesse sous le nom de marque NEOSORB^{®} à 99,5 % de matière sèche.

La préparation enzymatique présente alors une activité β-amylasique de 1400 °DP/ml.

### Exemple 5 : Obtention de sirops riches en maltose avec en témoin une β-amylase purifiée commerciale.

### a) obtention du sirop de maltose par l'action de la β-amylase seule

On réalise en laboratoire une saccharification comparée à l'aide d'une β-amylase purifiée commerciale de la société GENENCOR (OPTIMALT BBA) et à l'aide de la préparation de β-amylases conforme à l'invention, préparée selon l'exemple 1.

On ajoute la β-amylase, à la concentration de 4 ‰ (enzyme commerciale sur amidon sec), sur une solution d'amidon liquéfié à 30 % de matière sèche, présentant un taux de Dextrose Equivalant de 6 (mise en solution de maltodextrine de DE 6).

Les doses sont volontairement élevées pour détecter la présence éventuelle d'activités enzymatiques parasites.

La réaction est menée à une température de 58°C et à pH 5 pendant 24 heures.

Les résultats sont exprimés en % de monomères et d'oligomères de glucose générés par l'action β-amylasique, oligomères de degré de polymérisation égal à 2 et plus, déterminés par Chromatographie Liquide Haute Pression de type sodium.

Les DP2 correspondent au maltose, les DP1 au glucose et DP3 au maltotriose.

| | DP > 3 | DP3 | DP2 | DP1 |
|---|---|---|---|---|
| solution de départ (t = 0) | 96,5 | 1,5 | 1,2 | 0,3 |
| β-amylase GENENCOR (t = 24h) | 44 | 5,3 | 49,9 | 0,4 |
| β-amylase exemple 1 (t = 24h) | 42,6 | 5,7 | 51,4 | 0,4 |

Les résultats démontrent que la préparation de β-amylases conforme à l'invention peut se substituer efficacement la β-amylase commerciale.

### b) obtention d'un sirop riche en maltose par l'action d'une préparation β-amylasique combinée avec une pullulanase et une maltogénase en laboratoire.

Les essais sont réalisés dans les conditions précédente à la différence que l'on ajoute 2 enzymes simultanément à l'ajout de la préparation β-amylasique.

Il s'agit de la pullulanase PULLUZYME^{®} commercialisée par la société ABM qui hydrolyse spécifiquement les liaisons α 1→6 de l'amidon et de l'α-amylase maltogénique commercialisée par la société NOVOZYMES sous la dénomination MALTOGENASE^{®} qui hydrolyse spécifiquement les liaisons α 1→4.

Ces enzymes sont également ajoutées dans des proportions de 4 ‰ (enzyme commerciale sur amidon sec).

En témoin, on utilise comme source de β-amylases une préparation β-amylasique provenant d'un extrait de malt (= cocktail d'enzymes majoritairement composé de β-amylases).

| | DP SUP | DP3 | DP2 | DP1 |
|---|---|---|---|---|
| solution de départ (t = 0) | 96,5 | 1,5 | 1,2 | 0,3 |
| β-amylase GENENCOR (t = 24 h) | 7 | 2 | 85,3 | 5,5 |
| β-amylase selon l'exemple 1 (t = 24 h) | 7,2 | 1,5 | 85,5 | 5,2 |
| β-amylase extrait de malt T (t = 24 h) | 6,3 | 3,3 | 83,3 | 7,0 |

Si le profil glucidique obtenu à partir de la β-amylase purifiée ou de la préparation de β-amylases conformes à l'invention sont équivalents, démontrant par la même l'excellent comportement de la dite préparation selon l'invention, celui obtenu à partir d'extrait de malt produit une teneur en glucose bien supérieur.

### c) obtention d'un sirop riche en maltose par l'action d'une préparation β-amylasique combinée avec une pullulanase et une maltogénase à l'échelle industrielle.

Pour réaliser l'essai industriel, on utilise la préparation de l'exemple n°3.

La saccharification est réalisée sur 200 m³ d'une solution d'amidon liquéfié à 30 % de matière sèche, présentant un taux de Dextrose Equivalant de 6. La préparation de β-amylase (1 ‰ sur sec), la pullulanase et la maltogénase sont ajoutées simultanément dans les conditions suivantes : pH 5, 55 °C.

On obtient les résultats suivants après 66 heures (exprimés en % de DP générés - mesure effectuée par CLPHP sodium) :

| DP > 3 | DP3 | DP2 | DP1 |
|---|---|---|---|
| 6 | 1,1 | 88,3 | 4,3 |

Ce sirop riche en maltose peut être purifié, hydrogéné et cristallisé pour obtenir un maltitol de qualité identique celui obtenu par les voies classiques.

### d) obtention du sirop de maltose par l'action de la préparation de β-amylase ultrafiltrée et diafiltrée.

On réalise en laboratoire une saccharification comparée à l'aide de la préparation de β-amylases conforme à l'invention, préparée selon l'exemple 1 (β-amylase exemple 1), et à l'aide d'une préparation de β-amylases clarifiée selon l'exemple 1 mais non soumise aux étapes d'ultrafiltration et de diafiltration (β-amylase sans UF).

Pour la saccharification avec la préparation « β-amylase exemple 1 », on ajoute ladite préparation « β-amylase exemple 1 », à la concentration de 4 ‰ (enzyme commerciale sur amidon sec), sur une solution d'amidon liquéfié à 30% de matière sèche, présentant un taux de Dextrose Equivalent de 6 (mise en solution de maltodextrine de DE 6). La préparation enzymatique présente alors une activité β-amylasique de 1,8 °DP/ml. La réaction est menée à une température de 58°C et à pH 5 pendant 24h.

Pour la saccharification avec la préparation « β-amylase sans UF », on prend 11 de filtrat à 8 NTU de l'exemple 1 (filtrat non ultrafiltré ni diafiltré). On dilue ledit filtrat à 8 NTU au 1/15^{ème} de manière à obtenir une concentration de β-amylase à 1,8 °DP/ml. On ajuste le pH à 5. On prend 700 ml dudit filtrat dilué et on ajoute 300g de maltodextrine de DE 6. La réaction est menée à une température de 58°C pendant 24h.

On obtient les résultats suivants (exprimés en % de DP générés - mesure effectuée par CLPHP sodium) :

| | DP > 3 | DP3 | DP2 | DP1 |
|---|---|---|---|---|
| solution de départ (t = 0) | 96,5 | 1,5 | 1,2 | 0,3 |
| β-amylase sans UF (t = 24h) | 41,5 | 3,8 | 48,5 | 2,2 |
| β-amylase exemple 1 (t = 24h) | 42,6 | 5,7 | 51,4 | 0,4 |

Les résultats démontrent que la préparation de β-amylases non ultrafiltrée et non diafiltrée (β-amylase sans UF) permet d'obtenir un sirop riche en maltose contenant 2,2% de glucose. Contrairement à la préparation « β-amylase exemple 1 » (sirop riche en maltose obtenu contenant 0,4% de glucose, la préparation « β-amylase sans UF » est donc contaminée par des impuretés enzymatiques.

Afin d'évaluer le degré de pureté des sirops riches en maltose obtenus à l'aide des deux préparations « β-amylase exemple 1 » et « β-amylase sans UF », en particulier la quantité de sels qu'ils contiennent, la conductivité desdits sirops a été mesurée à 20°C (appareil CDM210 commercialisé par la société Mettler) après filtration des sirops sur un filtre pastille de porosité 1 µm de la société Millipore.

Les résultats sont les suivants :

| | Conductivité (µS) |
|---|---|
| β-amylase sans UF | 200 |
| β-amylase exemple 1 | 20 |

La préparation ultrafiltrée et diafiltrée (« β-amylase exemple 1 ») permet d'obtenir un sirop riche en maltose qui contient une quantité de sels très inférieure à celui obtenu grâce à la préparation « β-amylase sans UF ». En conséquence, la purification (filtration, décoloration, déminéralisation) du sirop obtenu grâce à la préparation « β-amylase sans UF » sera plus difficile que celle du sirop obtenu grâce à la préparation (« β-amylase exemple 1 ».

## Revendications

1. Procédé d'obtention d'une préparation de β-amylases à partir de la fraction soluble des plantes amidonnières, **caractérisé en ce que** l'on :
a) choisit la fraction soluble des plantes amidonnières dans le groupe constitué des fractions solubles du blé, du pois, de la fève, de la fèverole, du riz, de l'orge, du seigle, du sarrasin, de la pomme de terre et de la patate douce,
- les solubles de blé ou d'orge proviennent du flux de séparation des amidons « B » résultant de la séparation de l'amidon dans le procédé de l'amidonnerie de blé humide ou d'orge respectivement,
- les solubles de pomme de terre sont obtenus par récupération de la fraction soluble issue de l'écrasage des pommes de terre en début d'extraction de la fécule,
- les solubles de pois, de fève, de féverole ou de riz résultent de l'eau de trempage du pois, des fèves, des féveroles ou du riz respectivement et sont récupérés avant l'écrasage et la séparation des différents constituants du pois, des fèves, des féveroles ou du riz respectivement,
b) effectue une clarification desdites fractions solubles de manière à en éliminer les substances insolubles et les colloïdes,
c) réalise une ultrafiltration desdites fractions solubles clarifiées de manière à obtenir un rétentat d'ultrafiltration contenant la β-amylase concentrée et un perméat d'ultrafiltration,
d) récupère la β-amylase concentrée ainsi obtenue.

2. Procédé selon la revendication 1 **caractérisé en ce qu'**on réalise l'étape c), et que, entre les étapes c) et d), on réalise une diafiltration dudit rétentat d'ultrafiltration contenant la β-amylase concentrée

3. Procédé selon la revendication 1 ou 2, **caractérisé en ce que** la clarification des solubles est effectué par filtration, au moyen d'une technique choisie dans le groupe constitué de la filtration frontale, de la filtration sous pression et de la filtration sous vide.

4. Procédé selon la revendication 3, **caractérisé en ce que** la filtration sous vide est effectuée à l'aide de filtres à bandes ou de filtres rotatifs.

5. Procédé selon la revendication 1 ou 2, **caractérisé en ce que** la clarification est effectuée par centrifugation.

6. Procédé selon la revendication 5, **caractérisé en ce que** la centrifugation est réalisée à l'aide d'une centrifugeuse à assiettes de type séparateur à buses ou de type auto-débourbeur, ou d'une centrifugeuse à assiettes de type séparateur à buses vortex haute performance.

7. Procédé la revendication 1 ou 2, **caractérisé en ce que** la clarification est effectuée par filtration tangentielle membranaire.

8. Procédé selon la revendication 7, **caractérisé en ce que** la filtration tangentielle membranaire est une microfiltration d'un seuil de coupure compris entre 0,1 à 1 µm.

9. Procédé selon la revendication 1 ou 2, **caractérisé en ce que** l'ultrafiltration est réalisée à l'aide de membrane présentant un seuil de coupure de 10000 Da à 50000 Da.

10. Procédé selon la revendication 9, **caractérisé en ce que** l'ultrafiltration est réalisée à l'aide de membrane présentant un seuil de coupure de 30000 Da.

11. Procédé selon l'une quelconque des revendications 1 à 10, **caractérisé en ce que** la fraction soluble est une fraction soluble de blé ou d'orge.

## Patentansprüche

1. Verfahren zur Gewinnung eines Präparates von β-Amylasen aus der löslichen Fraktion von Stärkepflanzen, **dadurch gekennzeichnet, dass**:
a) die lösliche Fraktion von Stärkepflanzen aus der Gruppe auswählt ist, die aus löslichen Fraktionen von Weizen, Erbsen, Saubohnen, Pferdebohnen, Reis, Gerste, Roggen, Buchweizen, Kartoffeln und Süßkartoffeln besteht,
- die löslichen Stoffe von Weizen oder Gerste aus dem Abtrennstrom von B-Stärken stammen, der aus der Abtrennung der Stärke im Verfahren zur Stärkegewinnung aus geweichtem Weizen beziehungsweise Gerste resultiert,
- die löslichen Stoffe der Kartoffel durch Rückgewinnung der löslichen Fraktion erhalten werden, die durch die Zerkleinerung der Kartoffeln zu Beginn der Extraktion der Stärke entsteht,
- die löslichen Stoffe von Erbsen, Saubohnen, Pferdebohnen beziehungsweise Reis aus dem Quellwasser von Erbsen, Saubohnen, Pferdebohnen beziehungsweise Reis resultieren und vor Zerkleinerung und Abtrennung der verschiedenen Bestandteile von Erbsen, Saubohnen, Pferdebohnen beziehungsweise Reis rückgewonnen werden,
b) eine Klärung besagter löslicher Fraktionen durchgeführt wird, um davon die unlöslichen Substanzen und Kolloide zu entfernen,
c) eine Ultrafiltration besagter geklärter löslicher Fraktionen durchgeführt wird, um ein Ultrafiltrationsretentat, das die konzentrierte β-Amylase enthält, und ein Ultrafiltrationspermeat zu erhalten,
d) die so erhaltene konzentrierte β-Amylase rückgewonnen wird.

2. Verfahren gemäß Anspruch 1, **dadurch gekennzeichnet, dass** der Schritt c) durchgeführt wird und dass zwischen den Schritten c) und d) eine Diafiltration besagten Ultrafiltrationsretentats durchgeführt wird, das die konzentrierte β-Amylase enthält.

3. Verfahren gemäß Anspruch 1 oder 2, **dadurch gekennzeichnet, dass** die Klärung der löslichen Stoffe durch Filtration mithilfe einer Technik durchgeführt wird, die aus der Gruppe ausgewählt ist, bestehend aus Frontalfiltration, Druckfiltration und Vakuumfiltration.

4. Verfahren gemäß Anspruch 3, **dadurch gekennzeichnet, dass** die Vakuumfiltration mithilfe von Bandfiltern oder Rotationsfiltern durchgeführt wird.

5. Das Verfahren gemäß Anspruch 1 oder 2, **dadurch gekennzeichnet, dass** die Klärung mittels Zentrifugation durchgeführt wird.

6. Verfahren gemäß Anspruch 5, **dadurch gekennzeichnet, dass** die Zentrifugation mithilfe einer Tellerzentrifuge des Düsenseparator-Typs oder des selbstreinigenden Typs oder einer Tellerzentrifuge des Hochleistungs-Wirbeldüsenseparator-Typs durchgeführt wird.

7. Verfahren gemäß Anspruch 1 oder 2, **dadurch gekennzeichnet, dass** die Klärung mittels tangentialer Membranfiltration durchgeführt wird.

8. Verfahren gemäß Anspruch 7, **dadurch gekennzeichnet, dass** die tangentiale Membranfiltration eine Mikrofiltration mit einer Ausschlussgrenze zwischen 0,1 bis 1 µm ist.

9. Verfahren gemäß Anspruch 1 oder 2, **dadurch gekennzeichnet, dass** die Ultrafiltration mithilfe einer Membran durchgeführt wird, die eine Ausschlussgrenze von 10000 Da bis 50000 Da aufweist.

10. Verfahren gemäß Anspruch 9, **dadurch gekennzeichnet, dass** die Ultrafiltration mithilfe einer Membran durchgeführt wird, die eine Ausschlussgrenze von 30000 Da aufweist.

11. Verfahren gemäß einem der Ansprüche 1 bis 10, **dadurch gekennzeichnet, dass** die lösliche Fraktion eine lösliche Fraktion von Weizen oder Gerste ist.

## Claims

1. A method for obtaining a preparation of β-amylases from the soluble fraction of starch plants, **characterized in that**:
a) the soluble fraction of starch plants is selected from the group consisting of the soluble fractions of wheat, pea, broad bean, horse bean, rice, barley, rye, buckwheat, sweet potato and potato,
- the wheat or barley solubles originate from the "B"-starch separation stream resulting from the separation of starch in the wet wheat or barley starch industry process, respectively,
- the potato solubles are obtained by recovery of the soluble fraction resulting from the tuber crushing at the beginning of starch extraction,
- the soluble fractions of pea, broad bean, horse bean or rice result from the steep water of pea, broad bean, horse bean or rice and are recovered before crushing and separation of the various constituents of pea, broad bean, horse bean or rice, respectively,
b) a clarification of said soluble fractions is carried out in such a way as to remove therefrom the insoluble substances and the colloids,
c) an ultrafiltration of said clarified soluble fractions is carried out in such a way as to obtain an ultrafiltration retentate containing the concentrated β-amylase and an ultrafiltration permeate,
d) the resulting concentrated β-amylase is recovered.

2. The method as claimed in claim 1, **characterized in that** step c) is carried out, and that, between steps c) and d), a diafiltration of said ultrafiltration retentate containing the concentrated β-amylase is carried out.

3. The method as claimed in claim 1 or 2, **characterized in that** the clarification of the solubles is carried out by filtration, by means of a technique selected from the group consisting of frontal filtration, filtration under pressure and vacuum filtration.

4. The method as claimed in claim 3, **characterized in that** the vacuum filtration is carried out using belt filters or rotary filters.

5. The method as claimed in claim 1 or 2, **characterized in that** the clarification is carried out by centrifugation.

6. The method as claimed in claim 5, **characterized in that** the centrifugation is carried out using a disk centrifuge of self-cleaning or nozzle separator type, or a disk centrifuge of high-performance vortex nozzle separator type.

7. The method as claimed in claim 1 or 2, **characterized in that** the clarification is carried out by tangential membrane filtration.

8. The method as claimed in claim 7, **characterized in that** the tangential membrane filtration is a microfiltration having a cut-off threshold of between 0.1 and 1 µm.

9. The method as claimed in claim 1 or 2, **characterized in that** the ultrafiltration is carried out using a membrane having a cut-off threshold of from 10 000 Da to 50 000 Da.

10. The method as claimed in claim 9, **characterized in that** the ultrafiltration is carried out using a membrane having a cut-off threshold of 30 000 Da.

11. The method as claimed in any one of claims 1 to 10, **characterized in that** the soluble fraction is a soluble fraction of wheat or barley.
